# EUROPEAN PATENT SPECIFICATION

(11) **EP 1 309 384 B1**
(45) Date of publication and mention of the grant of the patent: **19.01.2011**
(21) Application number: 01937789.4
(22) Date of filing: 29.05.2001
(51) Int. Cl.: B01D 29/00, B01D 29/44, B01D 36/00, A61M 1/36

(54) **BLOOD TREATMENT FILTERS AND BLOOD COLLECTION SYSTEMS**
BLUTFILTER UND BLUTAUFBEREITUNGSSYSTEME
FILTRES DE TRAITEMENT SANGUIN ET SYSTEMES DE COLLECTION DE SANG

(30) Priority: 26.05.2000 US 579590; 10.07.2000 JP 2000208736; 10.07.2000 JP 2000208737
(43) Date of publication of application: 14.05.2003
(73) Proprietor: Fenwal, Inc., Lake Zurich, IL 60047 (US); Asahi Kasei Medical Co., Ltd., Chiyoda-ku Tokyo 101-8101 (JP)
(72) Inventor: LYNN, Daniel, R., Spring Grove, IL 60081 (US); WONS, Allen, R., Antioch, IL 60002 (US); MESPREUVE, Luc, B-7800 Ath Lessines (BE); VANDENDAUL, Daniel, B-7870 Lessines (BE); SOUDANT, Gregory, B-7890 Wodeq Lessines (BE); MUI, Tat, C., Chicago, IL 60660 (US); KARLOVSKY, Daniel, M., Palatine, IL 60074 (US); MURPHEY, Randy, Pleasant Prairie, WI 53158 (US); CALHOUN, Daniel, R., Gurnee, IL 60031 (US); OKA, Shin-ichiroh, Oita-shi, Oita 870-0163 (JP); TSUJI, Michihiro, Oita 870-0304 (JP)
(74) Representative: Geary, Stephen
(86) International application number: PCT/US2001/017320
(87) International publication number: WO 2001/091880

(56) References cited:
- US-A- 5 547 591
- US-A- 5 591 337

## Description

### TECHNICAL FIELD

The present invention generally relates to blood collection and processing filters, systems and methods for reducing the presence of or eliminating undesirable components such as aggregates and white blood cells (leukocytes) from blood. More particularly it relates to a precise and disposable blood treatment filter and systems incorporating such filters for eliminating minute aggregates and white blood cells from whole blood preparations, red blood cell preparations, platelet preparations and plasma preparations for use in blood transfusions. The present invention further relates to a blood treatment filter which is most suitable to be subjected to centrifugation for separating each blood component, wherein the filter itself is subjected to centrifugation along with the blood-containing bag and other components of the blood filter system.

### BACKGROUND ART

Systems composed of multiple, interconnected plastic bags have met widespread use and acceptance in the collection, processing and storage of blood components. Using these systems, whole blood is collected and separated into its clinical components (typically red blood cells, platelets, and plasma). The components are individually stored and used to treat a multiplicity of specific conditions and diseased states.

Before storing blood components for later use, it is desirable to minimize the presence of impurities or other materials that can cause undesired side effects in the recipient. For example, because of possible febrile reactions, it is generally considered desirable to remove substantially all the leukocytes from blood components before transfusion or storage.

Filtration is conventionally used to accomplish leuko-reduction. Systems and methods for reducing the number of leukocytes by filtration in multiple blood bag configurations are described, e.g., in Stewart, U.S. Patent 4,997,577; Stewart et al., U.S. Patent 6,128,048; Johnson et al., U.S. Patent 5,180,504; and Bellotti et al., U.S. Patent 5,527.472.

Whole blood collected from a blood donor usually is not used for transfusion as such, but is separated into several components such as a red blood cell preparation, platelet preparation, plasma preparation and the like. Each component is stored and is used for transfusion thereafter. Since minute aggregates and white blood cells contained in these blood preparations cause various side effects after transfusion, these undesirable components have been often eliminated prior to transfusion. The need to eliminate white blood cells has become widely recognized in recent years, and some European countries legislate all blood preparations to be used for transfusion only after applying an elimination treatment of the white blood cells.

The most common method for eliminating white blood cells from the blood preparation involves treating the blood preparation with a white blood cell elimination filter. While the treatment of the blood preparation using such a filter has been usually applied at a recipient patient's bed side, it is now common to treat the blood before storage at the blood center to assure better quality control of the blood preparations after eliminating the white blood cells, and for improving efficiency of the white blood cell elimination treatment.

A blood collection and separation set or system is typically composed of two to four flexible bags, guide tubes for connecting the bags, an anticoagulant, a red blood cell preservation solution and blood collection needles for collecting the blood from a donor, separating the blood into its several usable components, and storing each blood component. A system called a "closed system" or an "integrated system", in which the white blood cell elimination filter is integrated into the blood separation set, is widely favored for eliminating the white blood cells prior to storage. Such a system is described, for example, in Japanese unexamined patent application publication no. 1-320064 and in WO 92/20428.

While a filter comprising a filter medium made of a non-woven fabric or a porous material packaged in a hard vessel such as polycarbonate has been widely used for conventional white blood cell elimination filters, it was a problem that a steam sterilization process that is widely used for sterilization of the blood separation set can be hardly applied for the filter as described above since the vessel's gas permeability is low or practically nil. The closed systems include a system in which the white cells are eliminated initially from the whole blood after collection, followed by removing the white cell elimination filter and then subjecting the remainder to centrifugation for separating each component, and a system in which the white blood cells are separated after separating the whole blood into plural blood components by centrifugation. In the latter case, however, the white blood cell elimination filter is subjected to centrifugation together with the blood collection and separation set, whereby the hard vessel may damage the bags and guide tubes or the hard vessel itself may be broken due to the stress caused by centrifugation.

For solving the foregoing problem, a flexible white blood cell elimination filter has been developed and deployed (EP 0526678 and Japanese unexamined patent application publication no. 11-216179), wherein the material for the filter housing has properties similar to or the same as those of the bag used in the blood collection and separation set besides being excellent in flexibility and steam permeability.

However, the filter element should be once welded to a sheet of a flexible frame, followed by welding the frame to a housing material, in the white blood cell elimination filters as described above thus giving rise to a problem in that the manufacturing process becomes complicated. It has been also a problem that a large portion of the starting material is wasted since an effective filtration port is ensured by punching the sheet inside of the frame.

Flexible white blood cell elimination filters not using a frame sheet are disclosed in Japanese unexamined patent application publication no. 7-267871 and in WO95/17236. However, in the case of application 7-267871, the filter elements of these filters are welded together at the periphery thereby forming a rigid plastic plate in this area. Because of this, there is a risk of damage to the port itself to breakage by the stress generated during the centrifugation as in the past filter device including a rigid plastic housing. The filter in the former patent publication 7-267871 also has some drawbacks, in that the risk of infection to medical workers or contamination of the blood preparation with bacteria could not be avoided when the welded part happens to be broken to cause leakage by mis-operation and tough handling during filtration operations or by the stress of centrifugation, because the outermost circumferential edges of the filter element is welded to the vessel material.

A method for reducing the risk of leakage is reported in the latter patent application (WO95/17236), wherein the outermost circumference of the filter element is welded to the vessel material to cover the edge of the filter element with the vessel material With this structure, if the weldings of the filter material and the vessel material are insufficient, it may not be easy to detect any leakage of the blood caused by such insufficient weldings. A white blood cell elimination filter is described in US-A-5591337. A filter according to this patent comprises a flexible housing having inlet and outlet ports which are separated from each other by a filter pad assembly. The housing of the filter includes a single, continuous seal, with a portion of the seal connecting the housing to the filter pad assembly and another portion of the seal connecting sheets of the housing to each other.

### OBJECTS OF THE INVENTION

It is an object of the present invention to provide a flexible blood processing filter without using a sheet type flexible frame, which eliminates the above drawbacks of complicated manufacturing process and increased loss of raw materials.

It is another object of the present invention to provide a blood filter whose welded part is hardly susceptible to break even when it is subjected to the centrifuge stress in the operation.

Accordingly, one object of the present invention is to provide a flexible blood treatment filter that with no use of a flexible frame to consequently avoiding a complicated manufacturing process or increase of loss of the starting materials.

Another object of the present invention is to provide a blood treatment filter that can prevent the medical workers from the risks of exposure to infections or the blood preparations from contamination with bacteria, even when the seal portions between the filter element and the vessel is broken to cause leakage by a mis-operation and rough handling during the filtration operation, and by stress during centrifugation.

A different object of the present invention is to provide a blood treatment filter having a construction capable of detecting, by inspection during the manufacturing process, the risk of deteriorating the white blood cell elimination function of the filter element in case the blood short-passes through the cracks and insufficiently welded portions without passing through the filter element as an in situ passage for the blood.

Yet another object of the present invention is to provide a blood collection system comprising a container and an interconnected filter, which can be handled and centrifuged as a unit without breakage of the filter or damage to the container.

### SUMMARY DISCLOSURE OF THE INVENTION

The objects of the present invention can be attained by providing a blood treatment filter as defined in the appended claims. This can be achieved by integrating the flexible vessel with the filter element in a first seal area, outside of which a second seal area is provided to integrate the inlet side flexible vessel with the outlet side flexible vessel and furthermore by providing a non-seal area between said first and second area, thereby enabling the foregoing three objects to be simultaneously attained.

The above mentioned first and second object are accomplished by integrating a flexible vessel with a filter element in a first seal area, and providing a second seal area to integrate the flexible container at a location radially outwardly spaced apart from the first seal area. The outer peripheral edge of the filter element thus extends a width of 2 to 25 mm between the first and second seal areas

The invention provides a blood collection system as defined in claim 1. The system comprises a container for holding blood, and a filter as defined in claim 1 communicating with the container. The .container and filter are mutually arranged for handling as a unit. The filter contains a fibrous filter medium housed within two flexible sheets of plastic. A first seal joins the sheets directly to the filter medium inboard of the peripheral edge of the filter medium, and a second seal joins the sheets outboard of the peripheral edge of the filter medium A region of the filter medium extends between the first and second seals to cushion contact with the filter housing during handling.

The present invention provides a blood treatment filter as defined in claim 1. Suitably the filter comprises a flexible vessel having an inlet and an outlet for the blood, and a sheet of filter medium for eliminating undesirable components from the blood, wherein the inlet and outlet of the blood are partitioned from one another with .a sheet of the filter, comprising: a first seal area formed by integrating the entire circumference in the vicinity of the periphery of the sheet of the filter element with the flexible vessel; a second seal area formed by integrating the inlet side flexible vessel with the outlet side flexible vessel over the entire outer circumference of the first seal area; and a non-seal area between the first seal area and the second seal area. Preferably, the non-seal area has a gap width of 1 to 30 mm.

Furthermore, the present invention relates to said blood treatment filter wherein the filter element comprises at least one filter element eliminating the white blood cells.

The filter element can comprise a first filter element for eliminating aggregates from the blood, a second filter element placed downstream from the first filter element for eliminating the white blood cells, and a third filter element disposed between the second filter element and the outlet side of the vessel for preventing the outlet side of the vessel and the second filter element from adhering to each other.

The first seal area can be integrated with the flexible vessel at least the entire circumference in the vicinity of the periphery of the filter element for removing white cells.

The present invention preferably provides that the edge of the filter element is present with a width of 2 to 25 mm over the entire circumference in the non-sealed area. The edge of the peripheral portion of the filter element present in the non-sealed area is sometimes called "protruding filter element" in the following description.

The portions of the filter element present in the non-sealed area is sometimes called "step-out portion of the filter element" in the following description.

Other features and advantages of the invention will become apparent upon review of the following description, drawings, and appended claims.

### BRIEF DESCRIPTION OF THE DRAWINGS

FIG. 1 is a schematic view of a blood collection and storage system that includes an integral flexible filter that removes leukocytes from red blood cells;
FIG. 2 is top view of the integral flexible filter that forms a part of the system shown in Fig. 1;
FIG. 3 is a side section view of the filter shown in Fig. 2, taken generally along line 3-3 in Fig. 2 an assembled perspective view, of the integral flexible filter shown in Fig. 2; and
FIG. 4 is an exploded perspective view of the filter shown in Fig. 2, showing the assembly of a molded port to the filter.
FIG. 5 shows an illustrative cross section of the blood treatment filter according to the present invention.
FIG. 6 shows one embodiment of the process for manufacturing the blood treatment filter according to the present invention.
FIG. 7 shows another embodiment of the process for manufacturing the blood treatment filter according to the present invention.

The invention is not limited to the details of the construction and the arrangements of parts set forth in the following description or shown in the drawings. The invention can be practiced in other embodiments and in various other ways. The terminology and phrases are used for description and should not be regarded as limiting.

### DESCRIPTION OF THE PREFERRED EMBODIMENTS

The present invention will now be described in detail by way of the following, nonlimiting examples.

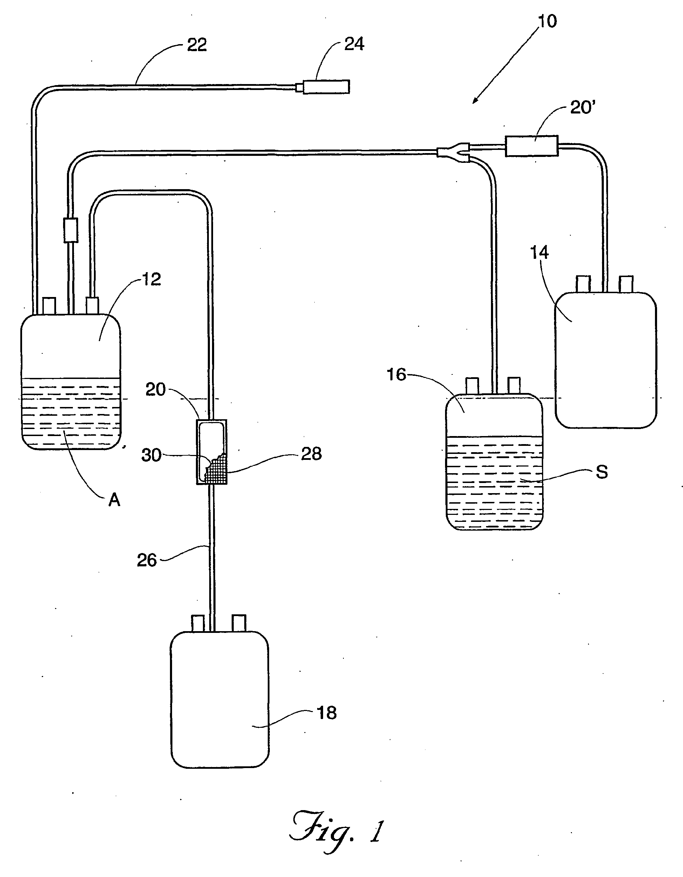

Fig. 1 shows a manual blood collection and storage system 10 having an integral flexible filter 20, which are arranged for handling as a unit. The system 10 provides red blood cells for long term storage that are substantially free of leukocytes. The system 10 also provides platelet concentrate and the platelet-poor plasma for long term storage. The blood collection and storage assembly 10, once sterilized, constitutes a sterile, "closed" system, as judged by the applicable standards in the United States. The system 10 is a disposable, single-use item.

As shown in Fig. 1, the system 10 includes a primary bag 12 and three transfer bags or containers 14,16, and 18. Like the flexible filter 20, the transfer bags 14, 16, and 18 are integrally attached to the system 10.

In use, the system 10 is handled in conventional ways. The primary bag 12 (which is also called a donor bag) receives whole blood from a donor through integrally attached donor tube 22 that carries phlebotomy needle 24. A suitable anticoagulant A is contained in the primary bag 12. The system 10, with attached filter 20, is disposed into a bucket of a centrifuge (not shown). The entire system 10, with attached filter, is spun within the centrifuge bucket. Whole blood is centrifugally separated inside the primary bag 12 into red blood cells and platelet-rich plasma. Leukocytes dwell in the interface between the red blood cells and platelet-rich plasma.

The transfer bag 14 is intended to receive platelet-rich plasma separated from the whole blood collected in the primary bag 12. Attempts are made when transferring the platelet-rich plasma out of the primary bag 12 to keep as many leukocytes in the primary bag 12 as possible. The transfer of platelet-rich plasma into the transfer bag 14 leaves the red blood cells and the leukocytes behind in the primary bag 12.

The transfer bag 16 contains a suitable storage solution S for red blood cells. One such solution is disclosed in Grode at al U.S. Patent 4,267,269, which is sold by Baxter Healthcare Corporation under the brand name ADSOL® Solution. The storage solution S is transferred into the primary bag 12 after transfer of the platelet-rich plasma into the transfer bag 14.

The platelet-rich plasma is centrifugally separated by conventional means in the transfer bag 14 into platelet concentrate and platelet-poor plasma. The platelet-poor plasma is transferred into the transfer bag 16, which is now emptied of storage solution S. The transfer bag 16 serves as the storage container for the platelet-poor plasma. The transfer bag 14 serves as a storage container for the platelet concentrate.

The storage solution S is mixed with the red blood cells and leukocytes remaining in the primary bag 12. The mixture of storage solution S, red blood cells, and leukocytes is transferred from the primary bag 12 through tubing 26.

The tubing 26 carries in-line the integral, flexible filter 20. The flexible filter 20 includes a filtration medium 28 contained within a housing 30. The filtration medium is selected to remove leukocytes from red blood cells. The filter 20, being flexible, facilitates handling and reduces the incidence of damage to other flexible plastic components of the system 10 during centrifugal processing.

The leukocyte-reduced red blood cells enter the transfer bag 18. The transfer bag 18 serves as the storage container for the leukocyte-reduced red blood cells.

The bags and tubing associated with the processing system 10 can all be made from conventional approved medical grade plastic materials, such as polyvinyl chloride plasticized with di-2-ethylhexylphthalate (PVC-DEHP). The bags are formed using conventional heat-sealing technologies, e.g., radio frequency (RE) heat sealing.

Alternatively, since the transfer bag 14 is intended to store the platelet concentrate, it can be made ofpolyolefin material (as disclosed in Gajewski et al U.S. Patent 4,140,162) or a polyvinyl chloride material plasticized with tri-2-ethylhexyl trimellitate (TEHTM). These materials, when compared to DEHP-plasticized polyvinyl chloride materials, have greater gas permeability that is beneficial for platelet storage.

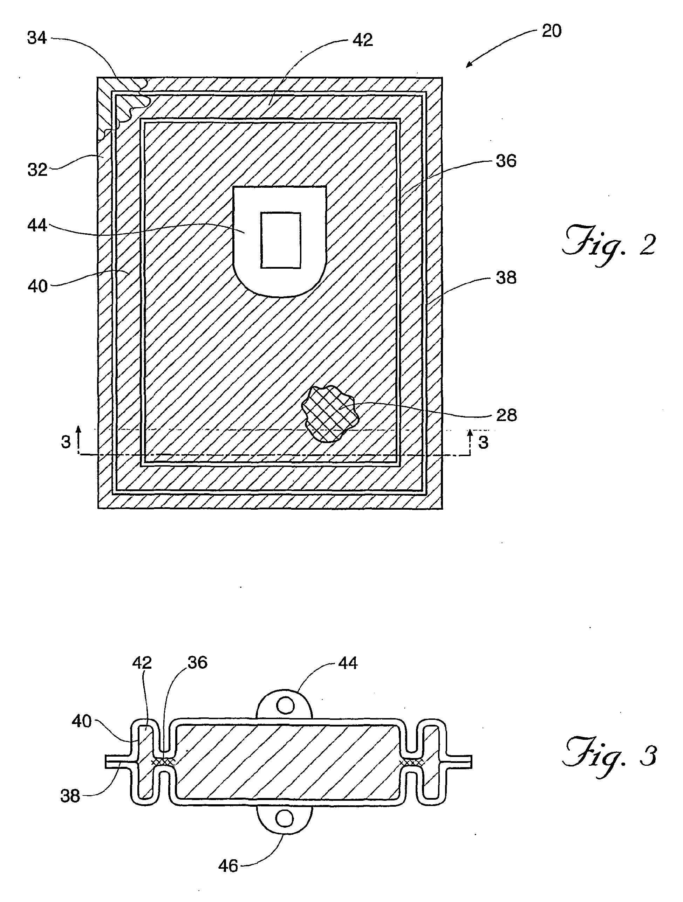

As shown in Fig. 2, the flexible filter 20 includes a filter housing 30 (see Fig. 1) comprising first and second sheets 32 and 34 of flexible, medical grade plastic material, such as polyvinyl chloride plasticized with di-2-ethylhexylphthalate (PVC-DEHP). Other medical grade plastic materials can be used that are not PVC and/or are DEHP-free.

The filtration medium 28 is made from a fibrous material, which is sandwiched between the sheets 32 and 34. The filtration medium 28 can be arranged in a single layer or in a multiple-layer stack. The medium 28 can include melt-blown or spun-bonded synthetic fibers (e.g., nylon or polyester or polyethylene or polypropylene), semi-synthetic fibers, regenerated fibers, or inorganic fibers. In use, the medium 28 removes leukocytes by depth filtration.

According to the invention (see Figs. 2 and 3) the filter 20 includes an inboard main seal 36 and an outboard secondary seal 38. The main seal 36 joins the two sheets 32 and 34 to each other, as well as joins the filtration medium 28 to the two sheets 32 and 34. The secondary seal 38 is spaced outboard of the peripheral edge 40 of the filtration medium 28 and joins just the two sheets 32 and 34 to each other.

As a result of this construction, a region 42 of filtration medium 28 extends between the main seal 36 and the secondary seal 38. The region 42 provides a "soft" periphery or "cushion" about the filter 20. The cushioned periphery provides enhanced protection against damage to tubing and bags of the system 10 when the bags, tubing and filter are handled as a unit, e.g., when centrifuged in the same centrifuge bucket. The combination of the main seal 36 inboard of the filtration medium 28 and the secondary seal 38 outboard of the filtration medium 28 also prevents edge flow and provides duplicate seal protection against leaks.

The main seal 36 can be formed by the application of pressure and radio-frequency heating to the two sheets 32 and 34 and filtration medium 28. The secondary seal 38 can likewise be formed by the application of pressure and radio-frequency heating to the two sheets 32 and 34. The main seal 36 and secondary seal 38 can be formed in sequential heat sealing processes, or simultaneously in a single heat-sealing process.

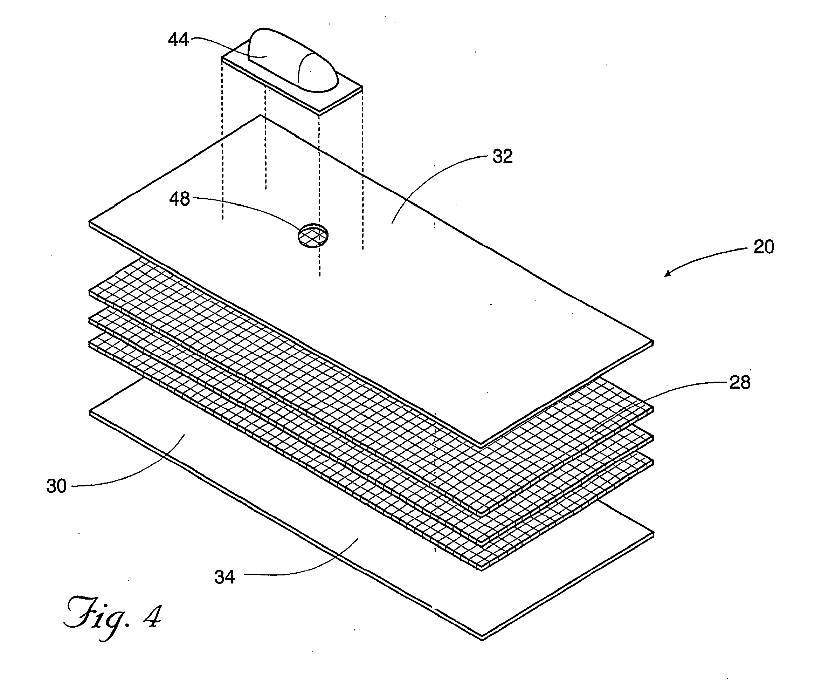

The filter 20 also includes inlet and outlet ports 44 and 46. As Fig. 4 shows, the ports 44 and 46 comprise separately molded parts that are heat sealed by radio-frequency energy over a hole 48 formed in the sheets 32 and 34, preferably before the main seal 36 and secondary seal 38 are created.

A flexible filter 20' (shown in phantom lines in Fig. 1) can be also be integrated into a multiple blood bag system in-line between the primary bag 12 and the transfer bag 14. In this arrangement, the filtration medium 28 is selected to remove leukocytes from platelet-poor plasma prior to entering the transfer bag 14.

While the overall configuration of the blood treatment filter according to the present invention may be any shape (e.g., rectangular, disk, or elliptical), the rectangular shape is preferable for reducing material loss in manufacturing the filter.

The flexible vessel to be used in the present invention is advantageously manufactured from a molded sheet or cylinder of a flexible synthetic resin, preferably a thermoplastic resin. The vessel may be manufactured by injection molding as an integrated molded body provided with an inlet or outlet of the blood.

Otherwise, holes or slits may be formed on a sheet or cylinder of a molded film manufactured by extrusion molding, to which independently molded inlet and outlet parts are connected such as by using an adhesive, heat-seal or high-frequency welding, so that the vessel and the inlet/outlet communicate with one another in a fluid-tight manner. However, the latter method is preferable since the vessel is hardly deformed by steam sterilization.

The material of the inlet/outlet ports can be the same as or different from the material of the molded film. Although the material is not particularly restricted so far as the inlet and the outlet are capable of being joined to the molded film with no gaps and to be fluid-tight besides causing no troubles in handling, the material of the inlet and outlet preferably has similar thermal and electrical properties to those of the molded film, because heat-sealing and high-frequency welding methods are advantageously used for mass-production. A suitable joining is possible by the high-frequency welding method when the molded film, the inlet, and the outlet are all made of materials having a relatively high dielectric constant such as soft polyvinyl chloride are welded one another, while the materials having a relatively low dielectric constant and melting point such as polyolefin can be favorably joined by heat-seal.

It is desirable that the flexible vessel have similar thermal and electrical properties to those of the filter element, examples of them being soft polyvinyl chloride, polyurethane, an ethylene-vinyl acetate copolymer, polyolefins such as polyethylene and polypropylene; thermoplastic elastomers such as a hydrogenated styrene-butadine-styrene copolymer, and a styrene-isoprene-styrene copolymer or its hydrogenated product; and mixed products of a thermoplastic elastomer and a softening agent such as polyolefin and ethylene-ethyl acrylate. Preferred examples include soft polyvinyl chloride, polyurethane, an ethylene-vinyl acetate copolymer and polyolefin, and a thermoplastic elastomer mainly comprising these polymers, and more preferable examples include soft polyvinyl chloride and polyolefin.

While many materials are available for the sheet of the filter element according to the present invention so far as it can eliminate undesirable components from the blood, such materials preferably are able to eliminate white blood cells. More preferably, the filter element according to the present invention includes a first filter element for eliminating aggregates from the blood at the inlet side, a second filter element for eliminating the white blood cells, and a third filter element disposed to prevent adhesion of the second element to the outlet side of the vessel.

Filtration media known in the art such as a fibrous and porous media include non-woven fabric and a porous medium having continuous pores of a three-dimensional network may be used for the filter element to be used in the present invention. Examples of these material include polypropylene, polyethylene, a styrene-isobutylene-styrene copolymer, polyurethane and polyester.

A combination of the filter elements having different fiber diameters and pore sizes is usually used.

In the case of the filter element comprising first, second and third filter elements, a filter material having a fiber diameter of several to several tens microns is disposed as the first filter element for eliminating aggregates, a filter material having a fiber diameter of 0.3 to 3.0 µm is disposed as the second filter element for eliminating the white blood cells, and a filter material having a fiber diameter of several to several tens microns is overlaid as the third filter element between the second filter element and the outlet side vessel for preventing the outlet side vessel from adhering to the second filter element.

The first, second and third filter elements can each be composed of a plurality of filter elements, respectively. It is preferable in this case that these filter elements are disposed so that the fiber diameter increases in gradation or continuously from the central portion of the second element having the first fiber diameter toward the inlet and the outlet.

It is also preferable that the filter elements are disposed so that the pore size increases in gradation or continuously from the central portion of the second element having the finest pore size toward the outlet and inlet, when porous materials having a three dimensional network of the continuous fine pores are used.

When the first seal area is formed, or the flexible vessel is joined to the inner part of inner part of the periphery of the edge of the filter element, inside welding such as high-frequency welding and ultrasonic welding or outside welding such as heat seal, or adhesion using a potting material may be employed. The high-frequency welding method is preferable when both of the flexible vessel and the filter element are formed of a material having a relatively high dielectric constant, and the heat seal method is preferable when one of the materials has a low dielectric constant, and both have a low melting point.

Although the first seal area may be formed either by a two-step welding method by which, after once welding the vicinity of the peripheral portion of the filter element, the portion is welded to the flexible vessel, or by a one step welding method by which the filter element is simultaneously welded to the flexible vessel, the one step welding method is preferable for simplifying the manufacturing process.

The first sealed area can be formed by either a two-step method, wherein the peripheral edge of the filter element is bonded inboard first and then the filter element and the flexible vessel are bonded together, or by a one-step method in which the filter element and the flexible vessel are bonded together simultaneously; however, the one-step bonding method is simpler and, therefore, preferable.

Although the first seal area can be formed either at the outermost peripheral portion of the filter element or at a point a little inside from the outermost peripheral portion, the latter method is particularly preferable when the seal area is formed by the high-frequency welding method or heat-seal method. In other words, it is preferable for stabilizing the manufacturing process that the non-sealed portion of the filter element extends to a width of several millimeters beyond the first seal area.

While the first seal area should not be necessarily formed by joining the entire filter element to the flexible vessel, it is necessary that at least the filter element for eliminating the white blood cells is welded to the flexible vessel, when the filter element includes the filter element for eliminating the white blood cells besides being laminated with other filter elements having different functions. This is because, when the filter element for eliminating the white blood cells is not integrated with the flexible vessel, the white blood cell elimination function can deteriorate due to short-pass.

Although the width of the first seal area is not particularly restricted, it is preferably within a range of 1 to 6 mm, more preferably 2 to 5 mm, in view of reliability and easy handling of the seal. When the width of the seal area is less than 1 mm, the joined portion becomes prone to failure of the sealing property during high-pressure sterilization or by rough handling. When the width is larger than 6 mm, on the other hand, the characteristics as the flexible vessel may be partly lost because the width of the sealing area portion that tends to be hardened by high-frequency welding, heat seal or impregnation of the potting agent turns out to be too large.

According to the present invention, it is essential that the first sealed area is formed inwardly from the position 2 to 25 mm away from the peripheral edge of the filter element so that the filter element holds a non-sealed part in the non-sealed area with a width of 2 to 25 mm over the entire circumference. When the blood treatment filer is subjected to centrifugation with the blood collection/separation set, this protruding filter element serves as a cushion so it prevents the blood treatment filter from damaging the blood bag of the blood collection/separation set and the circuit, as well as the blood treatment filter itself during centrifugation.

The way in which the blood processing filter can be damaged by centrifugation will now be described. There are many centrifugal cups of different forms, and the manner of placement of the separation/collection set and the blood processing filter in the centrifugal cup can vary depending on the form of particular cups. It is described here where the centrifugal cup is a cylindrical shape of one-liter capacity typically used in the United States.

In this centrifugal cup are placed a plasticized PVC bag formed of soft polyvinyl chloride containing about 570 ml of whole blood treated with anticoagulant, a blood processing filter, a bag formed of soft polyvinyl chloride containing about 100 ml of RBC storage solution and an empty bag for receiving the blood processed by the blood filter in the mentioned order, and all were subjected to centrifugation. Various conduits made of plasticized PVC connecting bags and the filter are disposed at an appropriate location between them.

When the system is centrifuged, various bags and the filter are forced against the bottom of the cup by centrifugal force. By this action, the bag containing whole blood and the bag containing RBC storage solution tend to deform and expand. As a result, the flexible blood treatment filter positioned between these two bags is pressed to collapse by the blood containing bag or molded to the shape of the expanded blood containing bag. As a result, the sealed portion between the filter element and the flexible vessel which has become a rigid plastic plate due to the welding, is bent, causing cracks or separation, resulting in leakage. When the sealed portion is pressed against the bottom of the centrifugal cup, such force causes cracks or leakage to the sealed portion.

On the other hand, in case of this invention, however, the presence protruding filter element serves as a cushion and decreases the amount of distortion caused in the sealed portion between the filter element and the flexible vessel, hence it protects the sealed portion when the filter is subjected to stress to deform or force against the cup bottom. Thus the protruding filter element remarkably reduces risks of damages. Furthermore, the protruding filter element helps to prevent direct contact of said sealed portion which has become a rigid plastic plate with the adjacent blood bag and conduits in the centrifugal cup, hence it prevents blood bag and conduit from being damaged.

With a width of this step-out portion less than 2 mm, a satisfactory effect cannot be expected. Conversely, when it is shaped to be disposed in aforementioned typical centrifugal cup, with a width in excess of 25 mm, even though it does not affect its efficiency, the width of the protruding element occupies two thirds of the filter width, providing no filter effect. Having an excess protruding element width is not practical. 2 mm or greater is enough as a width of said protruding filter element, however from the view point of mass production, the preferable width is 3 mm or greater, more preferably 4 mm or greater, and most preferably 5 mm or greater.

The upper limit of this width is 25 mm to be practical as already stated. However, this width is preferably 20 mm or less, more preferably 16 mm or less and most preferably 10 mm or less from the view point of the leak inspection duration.

The width of the protruding filter element is preferably uniform throughout the entire periphery. The deviation of this width is preferably 3 mm or less, more preferably 2 mm or less and most preferably 1 mm or less. Having a wide gap between the greatest width and the smallest width is not desirable, since the centrifugation stress tend to gather at the portion having the smallest width.

Although the entire filter elements need not be bonded to the flexible vessel, at least a layer for eliminating WBC must be bonded to the flexible vessel in the first seal area when the filter element consists of a plurality *of layers having different functions. Similarly, the protruding filter element may consist of either all of the elements constituting the effective area of the filter element or a portion thereof. As long as the expected cushion effect is achieved, either structure is acceptable. However, from the view point of simplifying the manufacturing process, it is more preferable if the protruding filter element constitutes all filter elements of the effective filtering portion.

The preferable width of the first seal area is within a range of 2 to 7 mm. When the width of the seal area is less than 2 mm, the joined portion assumes a line to fail the sealing property during high-pressure sterilization or by rough handling. For the same reason, the preferable width of the first seal area is 2 mm or greater, and the most preferable width is 3 mm or greater from the view point of production stability. When the width is larger than 7 mm, on the other hand, it causes the risks of becoming fragile against bending or deformation during the centrifugation, deteriorating the protection property of the protruding filter element. For the same reason, the more preferable width of the first sealed area id 6 mm or less.

It is necessary that the second seal area is formed over the entire circumference at outside of the first seal area, and the inlet side flexible vessel is integrated with the outlet side flexible vessel. The structure described above protects medical workers from infection, as it prevents the blood preparation from being contaminated with bacteria, even when the first seal area is broken to cause leakage by mis-operation or rough handling of the filter, or by the stress of centrifugation.

The second seal area is formed by joining among respective flexible vessels. While they can be joined by methods including inner welding such as high-frequency welding and ultrasonic welding, outer welding such as heat-seal and adhesion by a solvent, the high-frequency welding method is preferable when the flexible vessel is made of a material having a relatively high dielectric constant, and the heat-seal method is preferable when the flexible vessel is made of a material having a relatively low dielectric constant and melting point.

The width of the second seal area is desirably 1 to 10 mm, preferably 2 to 5 mm. Reliability of the seal may not be sufficiently exhibited when the width is less than 1 mm, and the width of 10 mm or less is preferable because too much welding width causes increase of the amount of the consumed materials. Although the flexible vessel according to the present invention can be formed using either a sheet of a film or a cylindrical film. If the blood treatment filter is formed from a sheet of film, the filter element can be inserted between two sheets of the film or between the folded portion of the sheet of the film. When the first seal area is formed by inserting the filter element between the folded portion of the sheet of the film, the second object of the present invention can be attained by sealing only the three open portions without forming the second seal area over the entire circumference, in order to form the second seal area of the present invention which is also within the scope of the present invention. Alternatively, when the first seal area is formed by inserting the filter element into the inner side of the cylindrical film, the second object of the invention can be attained by only sealing the open two side edges without forming the second seal area over the entire circumference, which also falls within the scope of the present invention.

It is essential that a non-seal area surrounded by the flexible vessel, the first seal area and the second seal area is formed between the first seal area and the second seal area. The width of the non-seal area should be within a range of 1 to 30 mm. When the width is less than 1 mm, the area may be engaged with the filter element while the second seal area is adhered, besides making it difficult to detect the leakage at the first seal areas as will be described hereinafter. It is not practical, on the other hand, that the width exceeds 30 mm, because detection of leakage at the first seal area takes too long.

The inspection to determine whether the leakage portion is present in the first seal area is carried out, for example, by a leak inspection method conducted as follows:
Tubes are connected to the blood inlet side and the blood outlet side, respectively. The tube segment communicating with the filter outlet is closed with forceps and air is introduced at 0.02 MPa from the filter inlet. The filter is allowed to stand in the air for a predetermined period of time, more specifically 1 minute to 1 hour depending on the width of the non-scaled area. If leakage of air has occurred, the gap between two seal areas (area h) will expand. Therefore, the presence or absence of leakage is judged by visual inspection of this portion.

In the filter according the present invention, the inner pressure of the filter decreases when the first seal area experiences a leakage, since a gap of 1 to 30 mm is provided between the first seal area and the second seal area. It is preferable that the width of the non-seal area is 2 mm or more, preferably 4 mm or more, considering reliability and ease of the leak detection. A width of the non-seal area of 20 mm or less is also preferable from the view point of the efficiency of the leakage detection, and a width of 10 mm or less is more preferable.

If the weldings of the filter material and the vessel material are insufficient, leakage may not be detected by the method as described above in the filter disclosed in WO95/17236 in which the seal area at the edge of the filter element is covered by being welded with the vessel material, wherein the inner pressure of the filter does not change because the blood does not leak to outside of the filter, even when the leakage causes a short-pass of the blood at the seal area of the edge of the filter element.

It is essential that a non-seal area surrounded by the flexible vessel, the first sealed area and the second sealed area is formed between the first sealed area and the second sealed area. By providing said protruding filter element in above defined area, the cushion property takes effect. The width of the non-seal area is preferably greater than the width of the protruding filter element preferably by 1 mm or greater, more preferably 2 mm or greater. If the difference is less than 1 mm, it causes unfavorable appearance because a part of the protruding filter element crosses over the second sealed area side, and it may deteriorate the reliability of the second sealed area. Having the difference of 10 mm or greater is unnecessary and it makes the handling more difficult. The width of the non-sealed area is greater than that of the protruding filter element preferably by 2 - 5 mm, most preferably by 3 - 4 mm.

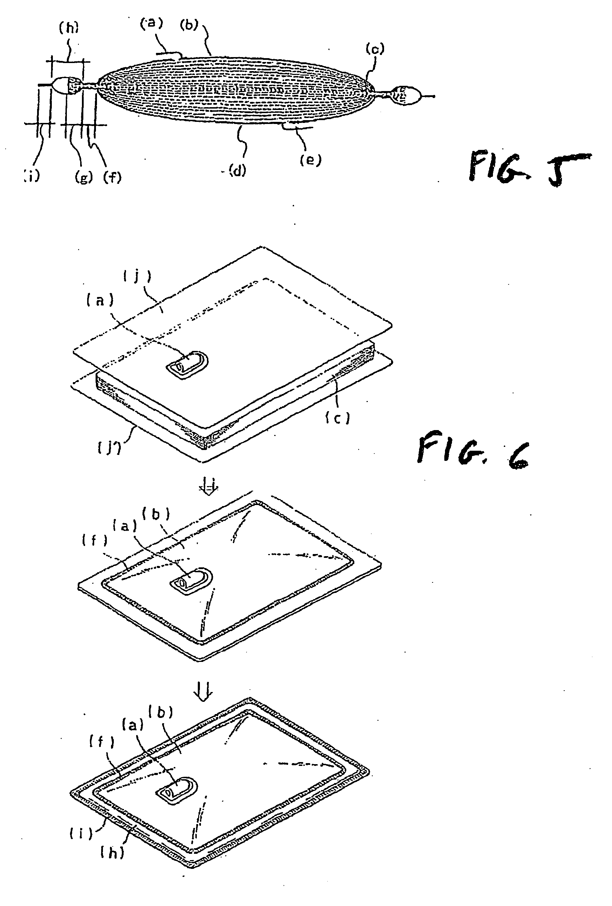

While an embodiment of the blood processing filter according to the present invention is shown in Fig. 5, the present invention is not restricted to this embodiment. The blood treatment filter is composed of an inlet side of the flexible vessel comprising a resin sheet b provided with a blood inlet a, an outlet side flexible vessel comprising a resin sheet d provided with a blood outlet e, the blood inlet a and the blood outlet e being partitioned with a filter element c. The filter element c of the filter is inserted between the inlet side flexible vessel and the outlet side flexible vessel, and the vicinity of the periphery of the filter element is integrated with the flexible vessel over the entire circumference of the element. A second seal area i, integrated by welding the inlet side flexible vessel and the outlet side flexible vessel, is formed at outside of the integrated first seal area f. The inlet side flexible vessel, the outlet side flexible vessel, and a non-sealed area h surrounded by the first seal area f and the second seal area i are provided between the first weal area f and the second sealed area i. A apart of the non-sealed filter element g protruding out of the first seal area f, when the first seal area f is formed at a little inside of the outermost circumference of the filter element c.

FIG. 6 shows one embodiment of the process of manufacturing the blood treatment filter according to the present invention. The non-seal area h is formed by inserting the filter element c between two sheets of the films j and j' on which the inlet a or the outlet is formed, by forming the first seal area f by heat-seal, and by further forming the second seal area i.

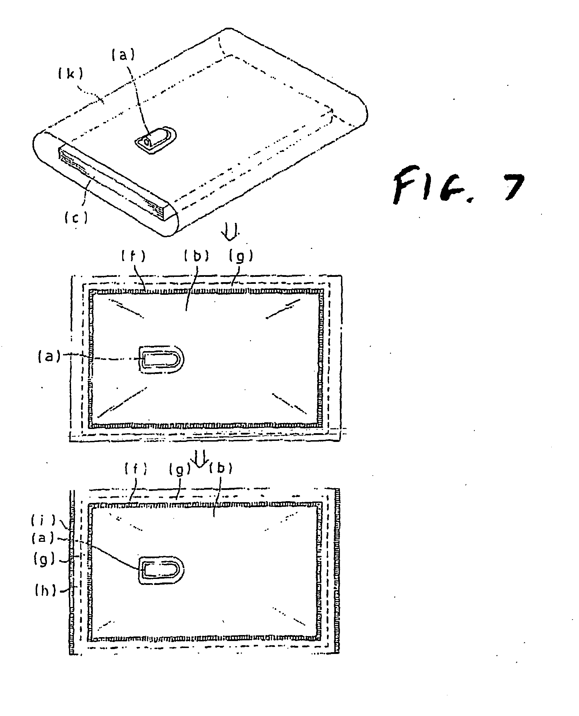

FIG. 7 shows an embodiment of another process for manufacturing the blood treatment vessel according to the present invention, wherein the flexible vessel is formed using a cylindrical film. The non-seal area h is formed by inserting the filter element c into a cylindrical film k on which the inlet a and the outlet are formed, by forming the first seal area f by heat-seal or other methods, and by further forming the second seal area i. The second seal area i may be merely formed at the open end.

While the white blood cell elimination filter according to the present invention will be described hereinafter in detail with reference to the examples, the present invention is not restricted by these examples. The method for inspecting leakage used in the examples and comparative examples is as follows.

### Method of Measurement

### (1) Sterilization and Centrifugation:

First prepared is an integral type system consisting of a blood treatment filter of the present invention, a blood collection bag A, a bag B for transferring PRP or blood plasma after being subjected to configuration, bag C containing about 100 ml of RBC storage solution, a bag D for receiving the blood components treated by the blood treatment filter after centrifugation, and various conduit connecting said parts wherein a blood collection tube is connected to the top of bag A. Then, another tube 2 is connected to the top of bag A, which is split via Y-shaped connector, and bags B and C are connected to each end. A third tube 8 extends from bag A to connect bag D via the blood filter disposed in between. The system was first sterilized by the high pressure steamed method at 121°C for 20 minutes. Then bag A is filled with 570 ml of CPD-anticoagulated bovine whole blood through tube 1. After tube 1 is sealed by the heat seal method at about 10 cm distant from bag A, the top portion is cut off. The system is disposed in a cylindrical centrifugal cup of about 1 liter capacity in the order of bag A, the blood treatment filter, bag C, bag D and bag B. Various conduits are disposed as needed between bags and blood treatment filter. The system is centrifuged using the device described below under the following conditions.
Centrifuge: CR783 (Hitachi Limited)
Radius of rotation: 0.261 m
Rotational Speed: 4140 rpm
Duration: 10 minutes
Cup size: 100 mm ID. 150 mm height

### (2) Leak inspection:

### (a) The leak inspection method of the blood filter sealed at only the first seal area

Tubes are connected to the blood inlet side and the blood outlet side, respectively. The outlet side tube is closed with a pair of forceps, and the air is injected from the blood inlet tube at a pressure of 0.02 MPa. The blood filter is kept under the water surface for a few minutes. Presence of leakage is judged by generation of air bubbles (named as an underwater leak inspection method hereinafter).

### (b) The leak inspection method of the blood filter sealed at the first and second seal areas.

Tubes are connected to the blood inlet side and the blood outlet side, respectively. The tube segment communicating with the filter outlet is closed with forceps and air is introduced at 0.02 MPa from the filter inlet. The filter is allowed to stand in the air for a predetermined period of time, more specifically 1 minute to 1 hour depending on the width of the non-scaled area. If leakage of air has occurred, the gap between two seal areas (area h) will expand. Therefore, the presence or absence of leakage is judged by visual inspection of this portion. Only those blood filters found to have no leakage in this inspection were used in the above system and subjected to the post sterilization and centrifugation leak inspection as above.

### Example 1

A flexible polyvinyl chloride resin sheets b and d, which were cut into a size of the blood filter plus 20 mm and on which holes were made at the portions corresponding to the blood inlet a and outlet e, and blood inlet a and blood outlet e, made of a polyvinyl chloride resin and formed by injection molding, were bonded by high-frequency welding one another, and the flexible vessel b provided with the blood inlet a, and the flexible vessel d provided with the blood outlet e were manufactured.

The polyester non-woven fabric to be described below was laminated for use as the filter element c. The following were laminated in the following order: Four sheets of the non-woven fabric 1 with a mean fiber size of 12 to 15 µm and fiber density of 29 to 31 g/m² for use as the first filter element, a total of twenty-seven sheets of the non-woven fabric comprising one sheet of the non-woven fabric 2 with a mean fiber size of 1.5 to 2.0 µm and fiber density of 65 to 67 g/m², 25 sheets of the non-woven fabric 3 with a mean fiber size of 1.2 to 1.4 µm and fiber density of 39 to 41 g/m², and one sheet of the non-woven fabric 2 for use as the second filter element, and four sheets of the non-woven fabric 1 for use as the third filter element. The first, second and third filter elements were laminated in this order, and the laminated body comprising in total 35 sheets of the non-woven fabric was out into a size of 85 mm X 68 mm (rectangle) for use as the filter element c. The flexible vessels b and d, and the filter element c were laminated in the order of the inlet side flexible vessel b, the filter element c and the outlet inlet side flexible vessel d. The first seal area was formed by high-frequency welding so that the filtration part has a size of 75x58 mm and the first seal area f has a width of 3 mm. The width of the protruding filter area g is 2 mm. High-frequency welding was purposely applied under a non-optimal condition for forming the first seal area so that leakage would happen with a certain frequency. The blood filters in which only the first seal area has been sealed were inspected according to the underwater inspection method, and the filters were classified into leaky filters and non-leaky filters.

Therefore, these filters were once dried, and the flexible vessels b and d were welded by high-frequency welding methods with the filters so that the width of the non-seal area h is 6 mm and the width of the second seal 1 is 3 mm. The final shape as shown in FIG. 5 was obtained by cutting the outermost periphery. Ten pieces each of the final shaped leaky filters and non-leaky filters were inspected by visual observation. The results are shown in TABLE 1.

The filter element (c) is lamination of the following three types of polyester nonwoven fabrics. The first filter element comprises 4 sheets of nonwoven fabric (1) having a fiber diameter of 12-15 µm and a fiber density of 29∼31 g/m⁴. The second filter element comprises 1 sheet of non-woven fabric (2) having a fiber size of 15∼20 µm, a fiber density of 65-67 g/cm², 25 sheets of nonwoven fabric (3) having a fiber diameter of 1.2-1.4 µm and a fiber density of 39-41 g/m⁴ and 1 sheet of nonwoven fabric. The third filter element comprises 4 sheets of nonwoven fabric (1). The laminate of nonwoven fabric sheets was cut in a rectangular shape (85 mm x 68 mm) and used as the filter element (c).

The flexible vessel halves (b, d) and the filter element (c) were overlaid as shown in Fig. 1 and RF welded together so as to give an effective filter area of 75 x 58 mm surrounded by the first seal area of 3 mm width. The width of protruding filter element (g) of the filter element was 1 mm or less.

In the next step, the second seal area having the width (i) of 3 mm was created between the flexible vessel halves (b, d) by RF welding so that the width of the nonsealed area (h) was 5 mm. The portions of flexible sheet outside the second seal area were cut off to give the desired filter assembly as shown in Fig. 1. The filters showing no leakage in the first seal area in the above leak test were sterilized and centrifuged as above and tested again for the presence of any leakage. The results are shown in Table 3.

### Example 2

The filter element c was cut into a size of 82 mm x 65 mm. The first seal area was welded so that the width of the protruding filter element g is 0.5 mm.

After subjecting the first seal portion to the leakage inspection, filters were manufactured by the same method as in Example 1, except that the flexible vessels b and d were welded so that the width of the non-seal area h is 1 mm. The results of the leakage test by the method described above are shown in TABLE 1.

### Example 3

Example 1 was followed except that the filter element was cut into 87 x 70 mm size so that the width of step-out portion (g) was 3 mm and the width of nonsealed area (h) was 6 mm. The difference between the greatest width and the smallest width of protruding filter element was 1 mm. The results of leak tests before and after sterilization/centrifugation are shown in Table 3.

### Example 4

Example 1 was followed except that the filter element was cut into 89 x 72 mm size so that the width of protruding filter element was 4 mm and the width of nonseal area (h) was 7 mm. The variation of the width of protruding element was 1 mm. The results of leak tests before and after sterilization/centrifugation are shown in Table 3.

### Example 5

The first seal area was welded to have a width of the protruding filter element g of 1 mm using the filter element c cut into a size of 83 mm x 66 mm. After a leakage inspection of the first seal portion, a filter was manufactured by the same method as in Example 1, except that the flexible vessels b and d were welded to have a width of the non-seal area h of 2 mm, and the filter was inspected with respect to leakage by the method described above. The results are shown in Table 1.

### Example 6

Example 1 was followed except that the filter element was cut into 91 x 74 mm size so that the width of protruding filter element was 5 mm and the width of nonseal area (h) was 8 mm. The variation of the width of step-out portion was less than 1 mm. The results of leak tests before and after sterilization/centrifugation are shown in Table 3.

### Example 7

A filter was manufactured by the same method as in Example 1, except that the flexible vessels b and d were welded to have a width of the non-seal area h of 4mm, and the filter was inspected with respect to leakage by the method described above. The results are shown in Table 1.

### Example 8

A filter was manufactured by the same method as in Example 1, except that the flexible vessels b and d were welded to have a width of the non-seal area h of 10 mm, and the filter was inspected with respect to leakage by the method described above. The results are shown in Table 1.

### Example 9

A filter was manufactured by the same method as in Example 1, except that the flexible vessels b and d were welded to have a width of the non-seal area h of 20 mm, and the filter was inspected with respect to leakage by the method described above. The results are shown in Table 1.

### Example 10

A filter was manufactured by the same method as in Example 1, except that the flexible vessels b and d were welded to have a width of the non-seal area h of 30 mm, and the filter was inspected with respect to leakage by the method described above. The results are shown in Table 1.

### Example 11

Example 1 was followed except that the filter element was cut into 97 x 80 mm size and assembled to a blood filter having an effective area of 71 x 54 mm, a step-out width (g) of 10 mm and a nonseal width (h) of 13 mm. The variation of the width of protruding filter element was 1 mm. The results of leak tests before and after sterilization/centrifugation are shown in Table 3.

### Example 12

Example 1 was followed except that the filter element was cut into 87 x 80 mm size in assembling to give a blood filter having an effective filter area of 51 x 44 mm, a protruding filter element width of 15 mm and a nonseal width of 18 mm. The variation of the step-out width (g) was 1 mm. The results of leak tests before and after sterilization/centrifugation are shown in Table 3.

### Example 13

Example 1 was followed except that the filter element was cut into 87 x 80 mm size in assembling to give a blood filter having an effective filter area of 31 x 24 mm and a nonseal width (h) of 28 mm. The variation of the step-out width (g) was 1 mm. The results of leak tests before and after the sterilization/centrifugation are shown in Table 3.

### Example 14

Example 6 was followed except that the filter element of 93 x 76 mm size was used to create the first seal area having a width of 4 mm. The variation of width was 1 mm. The results of leak test before and after the sterilization/centrifugation are shown in Table 3.

### Example 15

Example 6 was followed except that the filter clement of 97 x 80 mm size was used to create the first seal area having 6 mm width. The variation of the width was 1 mm. The results of leak tests before and after the sterilization/centrifugation are shown in Table 3.

### Example 16

Example 1 was followed except that the filter element cut into 83 x 66 mm size was used to create the first seal area of 2 mm width and the portion of 2 mm width. The variation of the step-out width was 1 mm. The results of leak tests before and after the sterilization/centrifugation are shown in Table 3.

### Example 17

Example 16 was followed except that the filter element cut into 93 x 76 mm size was used to create the first seal area having the width (f) of 2 mm width the width (g) of the filter element of 7 mm and the nonseal area width (h) of 10 mm. The variation of the step-out width was 1 mm. The results of leak tests before and after the sterilization/centrifugation are shown in Table 3.

### Example 18

Example 1 was followed except that the filter element cut into 97 x 80 mm size was used to create the first seal area of 7 mm width with the width (g) of 10 mm and the nonseal width (h) of 10 mm. The effective filter area was 63 x 46 mm. The variation of the width was 1 mm. The results of leak tests before and after the sterilization/centrifugation are shown in Table 3.

### Example 19

Example 18 was followed except that the filter element cut into 87 x 80 mm size was used to create the first seal area of 7 mm width with the width (g) of 15 mm and the nonseal width (h) of 18 mm. The effective filter area was 43 x 36 mm. The variation of the width was 1 mm. The results of leak tests before and after the sterilization/centrifugation are shown in Table 3.

### Example 20

Example 6 was followed to obtain a blood filter in which the variation of the width (g) was 2 mm. The results of leak tests before and after the sterilization/centrifugation are shown in Table 3.

### Example 21

Example 6 was followed to obtain a blood filter in which the variation of the width (g) was 3 mm. The results of leak tests before and after the sterilization/centrifugation are shown in Table 3.

### Comparative Example 1

Example 1 was followed except that the filter element cut into 83 x 66 mm size was used so that the width (g) was 1 mm and the nonseal area width (h) was 4 mm. The variation of the width (g) was 0.6 mm. The results of leak tests before and after the sterilization/centrifugation are shown in Table 4.

### Comparative Example 2

Example 1 was followed except that the filter element cut into 87 x 80 mm size was used to give an effective filter area of 25 x 18 mm and a width (g) of 28 mm in the nonseal area of 31 mm width. The variation of the width (g) was 1 mm. The results of leak tests before and after the sterilization/centrifugation are shown in Table 4.

### Comparative Example 3

The filter element o cut into a size of 81 mm x 64 mm was used, and the first seal area was welded so that the width of the protruding filter element g is zero mm. After leakage inspection of the first seal area, the filters were manufactured by the same method as in Example 1, except that the flexible vessels b and d were welded so that the width of the non-seal area h is zero mm.

However, a stable welding was difficult since sparks were often generated when the first seal area is subjected to high-frequency welding. It also happened that the end of the first seal area invaded into the second seal area to be unable to weld the second seal area. Although the filters not suffering these troubles were selected and subjected to the leakage inspection, it was impossible to discriminate the leaky filters from the non-leaky filters. The results of the visual inspection are shown in TABLE 2.

### Comparative Example 4

The filters was manufactured by the same method as in Example 2, except that the flexible vessels were welded so that the width of the non-seal area h of the blood filter is 0.5 mm. However, it happened that the end of the first seal area invaded into the second seal area to be unable to weld the second seal area. Although the filters not suffering these troubles were selected and subjected to the leakage inspection, it was difficult to discriminate the leaky filters from the non-leaky filters. The results of the visual inspection are shown in TABLE 2.

### Comparative Example 5

The filters were manufactured by the same method as in Comparative Example 1, except that the flexible vessels were welded so that the width of the non-seal area h of the blood filter is 0.5 mm. Stable welding was difficult as in Comparative example 1, besides discrimination of the leaky filters from the non-leaky filters by the visual inspection was difficult as in Comparative example 2. The results of the visual inspection are shown in TABLE 2.

### Comparative Example 6

The filters were manufactured by the same method as in Example 1, except that the filter element c cut to a size of 81.6 mm x 64.6 mm was used, and the first seal area was welded so that the width of the protruding filter element g is 0.3 mm. After subjecting the first seal portion to leakage inspection, the flexible vessels b and d were welded so that the width of the non-seal area h is 0.5 mm. The results of the leakage test by the same method as described above are shown in TABLE 2.

### Comparative Example 7

The filters were manufactured by the same method as in Example 1, except that the flexible vessels b and d were welded so that the width of the non seal area h of the blood filter is 35 mm. The results of the leakage test by the same method as described above are shown in TABLE 2.

**TABLE 1**

| | | Example 1 | Example 2 | Example 5 | Example 7 | Example 8 | Example 9 | Example 10 |
|---|---|---|---|---|---|---|---|---|
| Dimension of blood treatment filter (mm) | Vertical | 99 | 89 | 91 | 95 | 107 | 127 | 147 |
| | Horizontal | 82 | 72 | 74 | 78 | 90 | 110 | 130 |
| Dimension of filter element outermost circumference (mm) | Vertical | 85 | 82 | 83 | 85 | 85 | 85 | 85 |
| | Horizontal | 68 | 65 | 66 | 68 | 68 | 68 | 68 |
| Dimension of filtration part of blood filter (mm) | Vertical | 75 | 75 | 75 | 75 | 75 | 75 | 75 |
| | Horizontal | 58 | 58 | 68 | 58 | 58 | 58 | 58 |
| Width of first seal area | f (mm) | 3 | 3 | 3 | 3 | 3 | 3 | 3 |
| Width of protruding filter element | g (mm) | 2 | 0.5 | | 2 | 2 | 2 | 2 |
| Width of non-seal area | h (mm) | 6 | 1 | 2 | 4 | 10 | 20 | 30 |
| Width of second seal area | i (mm) | 3 | 3 | 3 | 3 | 3 | 3 | 3 |
| Leak detection ratio of first seal area of leaky filter | | 10/10 | 10/10 | 10/10 | 10/10 | 10/10 | 10/10 | 10/10 |
| Leak detection ratio of first seal area of non-leaky filter | | 0/10 | 0/10 | 0/10 | 0/10 | 0/10 | 0/10 | 0/10 |
| Leak detection time of first seal area of leaky filter (sec) | | 13 | 2 | 4 | 8 | 60 | 600 | 1800 |

**TABLE 2**

| | | Comparative example 3 | Comparative example 5 | Comparative example 5 | Comparative example 6 | Comparative example 7 |
|---|---|---|---|---|---|---|
| Dimension of blood treatment filter (mm) | Vertical | 87 | 88 | 88 | 88 | 157 |
| | Horizontal | 70 | 71 | 71 | 71 | 140 |
| Dimension of outer most circumference of filter (mm) | Vertical | 81 | 82 | 81 | 81.6 | 85 |
| | Horizontal | 64 | 65 | 64 | 64.6 | 68 |
| Dimension of filtration part of blood filter (mm) | Vertical | 75 | 75 | 75 | 75 | 75 |
| | Horizontal | 58 | 58 | 58 | 58 | 58 |
| Width of first seal area | :f (mm) | 3 | 3 | 3 | 3 | 3 |
| Width of filter material protruding from first seal area | :g (mm) | 0 | 0.5 | 0 | 0.3 | 2 |
| | | | | | | |
| Width of non-seal area between first and second seal areas | :h (mm) | 0 | 0.5 | 0.5 | 0.5 | 35 |
| Width of second seal area | :i (mm) | 3 | 3 | 3 | 3 | 3 |
| Leak detection ratio of first seal area of leaky filter | | 0/10 | 2/10 | 5/10 | 3/10 | 10/10 |
| Leak detection ratio of first seal area of non-leaky filter | | 0/10 | 0/10 | 0/10 | 0/10 | 0/10 |
| Leak detection time of first seal area of leaky filter (sec) | | - | 20 | 20 | 20 | 3600 |

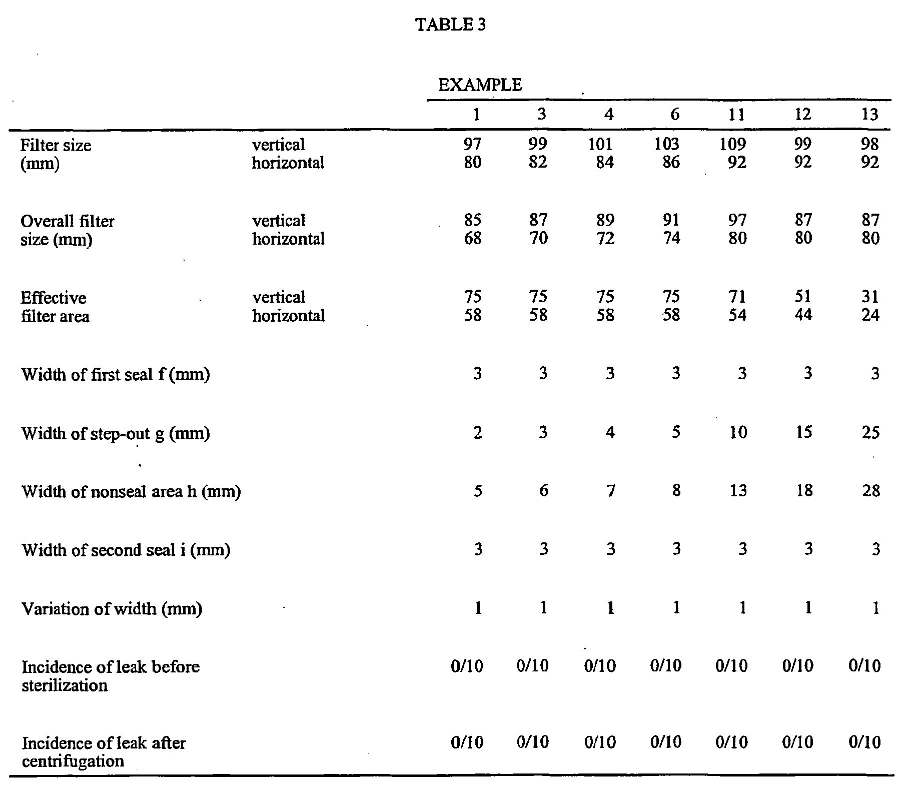

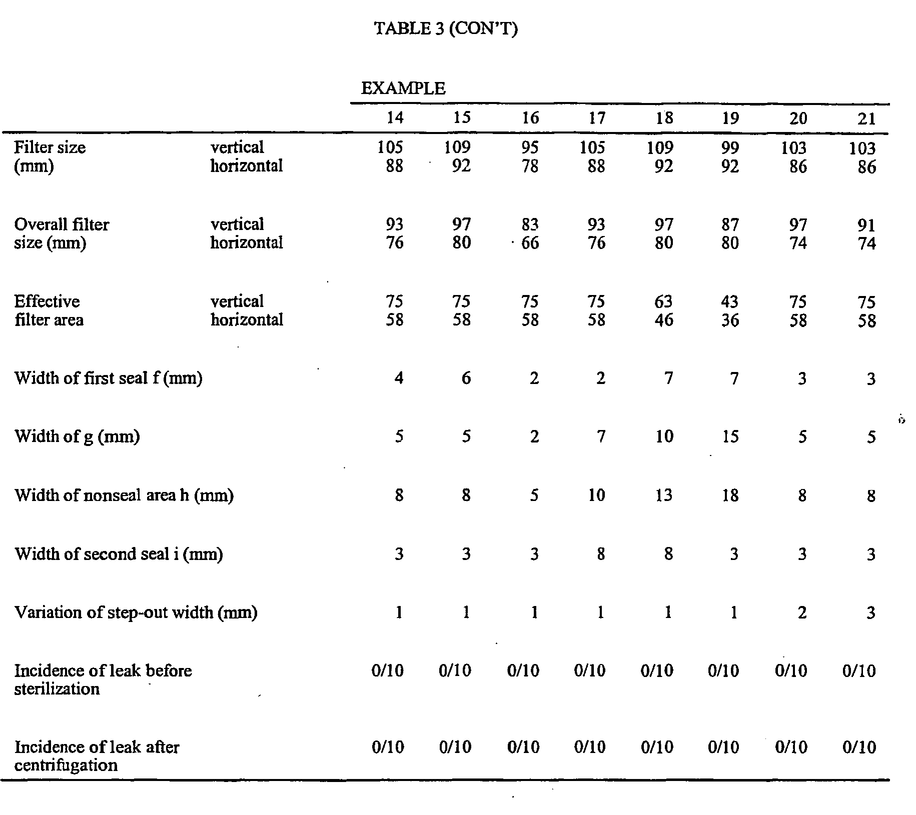

**TABLE4**

| | | COMPARATIVE EXAMPLE | |
|---|---|---|---|
| | | 1 | 2 |
| Filter size | vertical | 95 | 99 |
| (mm) | horizontal | 78 | 92 |
| | | | |
| Overall filter | vertical | 83 | 87 |
| size (mm) | horizontal | 66 | 80 |
| | | | |
| Effective | vertical | 75 | 25 |
| filter area | horizontal | 58 | 18 |
| | | | |
| Width of first seal f (mm) | | 3 | 3 |
| | | | |
| Width of g (mm) | | 1 | 28 |
| | | | |
| Width of nonseal area h (mm) | | 4 | 31 |
| | | | |
| Width of second seal i (mm) | | 8 | 8 |
| | | | |
| Variation of width (mm) | | 0.5 | 1 |
| | | | |
| Incidence of leak before sterilization | | 0/10 | 0/10 |
| | | | |
| Incidence of leak after centrifugation | | 4/10 | 0/10 |

According to the present invention, manufacturing of the flexible blood treatment filter is made possible without using any sheet type flexible frame. Even when the filter is subjected to the stress of centrifugation, and the welded portion is damaged, it does not deteriorate the white cell removing function. Furthermore, the present invention provides a blood treatment filter that protects the medical workers from the risk of infection and prevents the blood preparation from being contaminated by the bacterias.

According to the present invention, manufacturing of a flexible blood treatment filter is made possible without using any sheet of the flexible frame. In addition, the present invention provides the blood treatment filter that allows the medical workers to be protected from the risks of infections, and the blood preparation from being contaminated with bacteria and that is possible to detect by inspection the presence of cracks that may deteriorate the white blood cell elimination function of the filter.

## Claims

1. A blood treatment filter (20) comprising a flexible vessel (30) having an inlet (44) and an outlet (46), and a sheet of a filter element (28) for eliminating undesirable components from the blood,
wherein the inlet (44) and outlet (46) are partitioned by the filter element (28), comprising:
a first seal area (36) formed by integrating the entire circumference in the vicinity of the periphery (40) of the sheet of the filter element (28) with the flexible vessel (30); and
a second seal area (38) formed by integrating the inlet side (32) of the flexible vessel (30) with the outlet side (34) of the flexible vessel (30) over the entire outer circumference of the first seal area (36), and
a non-seal area between the first seal area (36) and the second seal area (38).

2. A blood treatment filter (20) according to claim 1, wherein the sheet of the filter element (28) comprises at least a filter element for eliminating white blood cells from the blood.

3. A blood treatment filter (20) according to any one of claims 1 to 2, wherein the first seal area (36) is integrated with the flexible vessel (30) over the entire circumference of the filter element (28) in the vicinity of the periphery (40) of the filter element (28).

4. A blood treatment filter (20) according to any one of claims 1 to 3, wherein the non-seal area has a gap width of 1 to 30 mm between the first seal area (36) and the second seal area (38).

5. A blood treatment filter (20) according to any one of claims 1 to 4, wherein the edge (42) of the filter element (28) is present in the non-seal area.

6. A blood treatment filter (20) according to claim 5, wherein the edge (42) of the filter element (28) present in the non-seal area has a width of 2 to 25 mm.

7. A blood treatment filter (20) according to claim 6, wherein the width of the edge (42) of the filter element (28) present in the non-seal area has a difference between a maximum width and a minimum width of 3 mm or smaller.

8. A blood treatment filter (20) according to any one of claims 1 to 7, wherein the filter element (28) comprises a first filter element for eliminating aggregates from the blood, a second filter element disposed downstream of the first filter element for eliminating white blood cells from the blood, and a third filter element disposed between the second filter element and the outlet side (34) of the flexible vessel (30) for preventing the outlet side (34) of the flexible vessel (30) and the second filter element from adhering.

9. A blood treatment filter (20) according to any one of claims 1 to 8, wherein the flexible vessel (30) is formed of a sheet of a molded body.

10. A blood treatment filter (20) according to any one of claims 1 to 9, wherein the flexible vessel (30) is formed of a cylindrical molded body.

11. A blood treatment filter (20) according to any one of claims 1 to 10, wherein the inlet (44) and outlet (46) are formed of molded members and are connected to the flexible vessel (30) to be liquid-tight.

12. A blood treatment filter (20) according to any one of claims 1 to 11, wherein the flexible vessel (30) is formed of soft polyvinyl chloride.

13. A blood treatment filter (20) according to any one of claims 1 to 11, wherein the flexible vessel (30) is formed of polyolefin.

14. A blood treatment filter (20) according to any one of claims 1 to 13, wherein at least one of the inlet and outlet (44, 46) is spaced from the first and second seal areas (36, 38).

15. A blood treatment filter (20) according to any one of claims 1 to 14, wherein the filter (20) is rectilinear in shape.

16. A blood treatment filter (20) according to any one of claims 1 to 15, wherein the filter (20) is rectangular in shape.

17. A blood collection system (10) comprising a container (12) for holding blood, and a filter (20) communicating with the container (12), the container (12) and filter (20) being mutually arranged for handling as a unit, wherein the filter (20) comprises a fibrous filter medium and is provided according to any one of claims 1 to 16.

18. A system (10) according to claim 17, wherein the filter (20) is integrally connected by tubing (26) to the container (12).

19. A system (10) according to any one of claims 17 to 18, wherein a second container (18) for receiving blood communicates with the filter (20), the first-defined container (12), the second container (18), and the filter (20) being mutually arranged for handling as a unit.

20. A system (10) according to claim 19, wherein the filter (20) is located in-line between the first-defined container (12) and the second container (18).

## Patentansprüche

1. Blutbehandlungsfilter (20), umfassend ein flexibles Behältnis (30) mit einem Einlass (44) und einem Auslass (46), und einer Bahn bzw. einem Bogen eines Filterlements (28) zum Eliminieren unerwünschter Komponenten aus dem Blut,
wobei der Einlass (44) und der Auslass (46) durch das Filterelement (28) getrennt sind, umfassend:
einen ersten Dichtungsbereich (36), der durch Integrieren des gesamten Umfangs in der Nähe der Peripherie (40) der Bahn des Filterelements (28) mit dem flexiblen Behältnis (30) gebildet ist; und
einen zweiten Dichtungsbereich (38), der durch Integrieren der Einlassseite (32) des flexiblen Behältnis (30) mit der Auslassseite (34) des flexiblen Behältnis (30) über den gesamten Außenumfang des ersten Dichtungsbereichs (36) gebildet ist, und
einen Nichtdichtungsbereich zwischen der ersten Dichtungsbereich (36) und dem zweiten Dichtungsbereich (38).

2. Blutbehandlungsfilter (20) nach Anspruch 1, wobei die Bahn des Filterelements (28) zumindest ein Filterelement zum Eliminieren weißer Blutkörperchen aus dem Blut umfasst.

3. Blutbehandlungsfilter (20) nach einem der Ansprüche 1 bis 2, wobei der erste Dichtungsbereich (36) mit dem flexiblen Behältnis (30) über den gesamten Umfang des Filterelements (28) in der Nähe der Peripherie (40) des Filterelements (28) integriert ist.

4. Blutbehandlungsfilter (20) nach einem der Ansprüche 1 bis 3, wobei der Nichtdichtungsbereich eine Spaltbreite von 1 bis 30 mm zwischen dem ersten Dichtungsbereich (36) und dem zweiten Dichtungsbereich (38) aufweist.

5. Blutbehandlungsfilter (20) nach einem der Ansprüche 1 bis 4, wobei die Kante bzw. der Rand (42) des Filterelements (28) in dem Nichtdichtungsbereich vorhanden ist.

6. Blutbehandlungsfilter (20) nach Anspruch 5, wobei der Rand (42) des Filterelements (28), der in dem Nichtdichtungsbereich vorhanden ist, eine Breite von 2 bis 25 mm aufweist.

7. Blutbehandlungsfilter (20) nach Anspruch 6, wobei die Breite des Rands (42) des Filterelements (28), der in dem Nichtdichtungsbereich vorhanden ist, eine Differenz zwischen einer maximalen Breite und einer minimalen Breite von 3 mm oder weniger aufweist.

8. Blutbehandlungsfilter (20) nach einem der Ansprüche 1 bis 7, wobei das Filterelement (28) ein erstes Filterelement zum Eliminieren von Aggregaten bzw. Anhäufungen aus dem Blut, ein zweites Filterelement, dass stromabwärts des ersten Filterelements angeordnet ist, um weiße Blutkörperchen aus dem Blut zu eliminieren, und ein drittes Filterelement umfasst, das zwischen dem zweiten Filterelement und der Auslassseite (34) des flexiblen Behältnis (30) angeordnet ist, um zu verhindern, dass die Auslassseite (34) des flexiblen Behältnis (30) und das zweite Filterelement aneinander haften.

9. Blutbehandlungsfilter (20) nach einem der Ansprüche 1 bis 8, wobei das flexible Behältnis (30) aus einer Bahn bzw. einem Bogen eines Formkörpers gebildet ist.

10. Blutbehandlungsfilter (20) nach einem der Ansprüche 1 bis 9, wobei das flexible Behältnis (30) aus einem zylindrischen Formkörper gebildet ist.

11. Blutbehandlungsfilter (20) nach einem der Ansprüche 1 bis 10, wobei der Einlass (44) und der Auslass (46) aus Formgliedern gebildet sind und mit dem flexiblen Behältnis (30) verbunden sind, um flüssigkeitsdicht zu sein.

12. Blutbehandlungsfilter (20) nach einem der Ansprüche 1 bis 11, wobei das flexible Behältnis (30) aus weichem Polyvinylchlorid gebildet ist.

13. Blutbehandlungsfilter (20) nach einem der Ansprüche 1 bis 11, wobei das flexible Behältnis (30) aus Polyolefin gebildet ist.

14. Blutbehandlungsfilter (20) nach einem der Ansprüche 1 bis 13, wobei zumindest einer des Einlass und des Auslass (44, 46) von dem ersten und dem zweiten Dichtungsbereich (36, 38) beabstandet ist.

15. Blutbehandlungsfilter (20) nach einem der Ansprüche 1 bis 14, wobei der Filter (20) eine geradlinige Form aufweist.

16. Blutbehandlungsfilter (20) nach einem der Ansprüche 1 bis 15, wobei der Filter (20) eine rechteckige Form aufweist.

17. Blutsammelsystem (10), umfassend einen Behälter (12) zum Halten von Blut und einen Filter (20), der mit dem Behälter (12) kommuniziert bzw. verbunden ist, wobei der Behälter (12) und der Filter (20) zum Handhaben als eine Einheit zueinander angeordnet sind, wobei der Filter (20) ein faseriges Filtermedium umfasst und gemäß einem der Ansprüche 1 bis 16 vorgesehen ist.

18. System (10) nach Anspruch 17, wobei der Filter (20) integral bzw. einstückig durch einen Schlauch (26) mit dem Behälter (12) verbunden ist.

19. System (10) nach einem der Ansprüche 17 bis 18, wobei ein zweiter Behälter (18) zum Aufnehmen von Blut mit dem Filter (20) kommuniziert bzw. verbunden ist, wobei der zuerst definierte Behälter (12), der zweite Behälter (18) und der Filter (20) zum Handhaben als eine Einheit zueinander angeordnet sind.

20. System (10) nach Anspruch 19, wobei der Filter (20) in-line zwischen dem zuerst definierten Behälter (12) und dem zweiten Behälter (18) angeordnet ist.

## Revendications

1. Filtre de traitement sanguin (20) comprenant un réservoir flexible (30) ayant une entrée (44) et une sortie (46), et une feuille d'un élément filtrant (28) pour éliminer les composants indésirables du sang,
dans lequel l'entrée (44) et la sortie (46) sont séparées par l'élément filtrant (28), comprenant
une première zone d'étanchéité (36) formée en intégrant toute la circonférence à proximité de la périphérie (40) de la feuille de l'élément filtrant (28), avec le réservoir flexible (30) ; et
une seconde zone d'étanchéité (38) formée en intégrant le côté entrée (32) du réservoir flexible (30) avec le côté sortie (34) du réservoir flexible (30) sur toute la circonférence extérieure de la première zone d'étanchéité (36) et
une zone de non étanchéité entre la première zone d'étanchéité (36) et la seconde zone d'étanchéité (38).

2. Filtre de traitement sanguin (20) selon la revendication 1, dans lequel la feuille de l'élément filtrant (28) comprend au moins un élément filtrant pour éliminer les leucocytes du sang.

3. Filtre de traitement sanguin (20) selon l'une quelconque des revendications 1 à 2, dans lequel la première zone d'étanchéité (36) est intégrée avec le réservoir flexible (30) sur toute la circonférence de l'élément filtrant (28) à proximité de la périphérie (40) de l'élément filtrant (28).

4. Filtre de traitement sanguin (20) selon l'une quelconque des revendications 1 à 3, dans lequel la zone de non-étanchéité a une largeur d'intervalle de 1 à 30 mm entre la première zone d'étanchéité (36) et la seconde zone d'étanchéité (38).

5. Filtre de traitement sanguin (20) selon l'une quelconque des revendications 1 à 4, dans lequel le bord (42) de l'élément filtrant (28) est présent dans la zone de non-étanchéité.

6. Filtre de traitement sanguin (20) selon la revendication 5, dans lequel le bord (42) de l'élément filtrant (28) présent dans la zone de non-étanchéité a une largeur de 2 à 25 mm.

7. Filtre de traitement sanguin (20) selon la revendication 6, dans lequel la largeur du bord (42) de l'élément filtrant (28) présent dans la zone de non-étanchéité a une différence entre une largeur maximale et une largeur minimale de 3 mm ou moins.

8. Filtre de traitement sanguin (20) selon l'une quelconque des revendications 1 à 7, dans lequel l'élément filtrant (26) comprend un premier élément filtrant pour éliminer des agrégats du sang, un second élément filtrant disposé en aval du premier élément filtrant pour éliminer les leucocytes du sang, et un troisième élément filtrant disposé entre le second élément filtrant et le côté sortie (34) du réservoir flexible (30) pour empêcher le côté sortie (34) du réservoir flexible (30) et le second élément filtrant d'adhérer.

9. Filtre de traitement sanguin (20) selon l'une quelconque des revendications 1 à 8, dans lequel le réservoir flexible (30) est formé d'une feuille d'un corps moulé.

10. Filtre de traitement sanguin (20) selon l'une quelconque des revendications 1 à 9, dans lequel le réservoir flexible (30) est formé d'un corps moulé cylindrique.

11. Filtre de traitement sanguin (20) selon l'une quelconque des revendications 1 à 10, dans lequel l'entrée (44) et la sortie (46) sont formées d'éléments moulés et sont raccordées au réservoir flexible (30) de façon à être étanches au liquide.

12. Filtre de traitement sanguin (20) selon l'une quelconque des revendications 1 à 11, dans lequel le réservoir flexible (30) est formé de chlorure de polyvinyle souple.

13. Filtre de traitement sanguin (20) selon l'une quelconque des revendications 1 à 11, dans lequel le réservoir flexible (30) est formé de polyoléfine.

14. Filtre de traitement sanguin (20) selon l'une quelconque des revendications 1 à 13, dans lequel au moins une parmi l'entrée et la sortie (44, 46) est espacée de la première et de la seconde zones d'étanchéité (36, 38).

15. Filtre de traitement sanguin (20) selon l'une quelconque des revendications 1 à 14, dans lequel le filtre (20) est de forme rectiligne.

16. Filtre de traitement sanguin (20) selon l'une quelconque des revendications 1 à 15, dans lequel le filtre (20) est de forme rectangulaire.

17. Système de collecte de sang (10) comprenant un récipient (12) pour contenir le sang, et un filtre (20) communiquant avec le récipient (12), le récipient (12) et le filtre (20) étant réciproquement disposés de façon à être manipulés comme une seule unité, dans lequel le filtre (20) comprend un moyen filtrant fibreux et est fourni selon l'une quelconque des revendications 1 à 16.

18. Système (10) selon la revendication 17, dans lequel le filtre (20) est intégralement raccordé par un tubage (26) au récipient (12).

19. Système (10) selon l'une quelconque des revendications 17 à 18, dans lequel un second récipient (18) pour recevoir du sang communique avec le filtre (20), le premier récipient défini (12), le second récipient (18) et le filtre (20) étant réciproquement disposés de façon à être manipulés comme une seule unité.

20. Système (10) selon la revendication 19, dans lequel le filtre (20) est aligné entre le premier récipient défini (12) et le second récipient (18).
